# DEMANDE DE BREVET EUROPEEN

(11) **EP 2 923 730 A1**
(43) Date de publication de la demande: **30.09.2015**
(21) Numéro de dépôt: 15160381.8
(22) Date de dépôt: 23.03.2015
(51) Int. Cl.: A61N 5/06, A61N 1/375, A61N 1/32, H01R 13/518, H01R 33/88, H05K 5/00, H05K 5/02, H05K 7/14, A61B 19/02

(54) **Interconnecteur structurel et électrique pour un équipement multi-modulaire**

(30) Priorité: 24.03.2014 FR 1452441; 20.11.2014 FR 1402619
(71) Demandeur: LUCIBEL, 92500 Rueil-Malmaison (FR)
(72) Inventeur: Houot, Jean-Laurent, 38110 Saint-Clair-de-la-Tour (FR)
(74) Mandataire: Martin, Marie-Aude

(57) **Abrégé**

L'interconnecteur structurel et électrique de deux modules entre eux d'un équipement multi-modulaire comprend des moyens de liaison (38A) et des moyens de liaison complémentaires (38B) aptes à coopérer ensemble pour former une liaison mécanique libérable des deux modules entre eux. Plus précisément, les moyens de liaison (38A, 38B) sont électriquement conducteurs pour autoriser le passage d'un courant électrique entre les deux modules et les moyens de liaison (38A, 38B) sont de type à aimantation pour former une liaison magnétique amovible entre les deux modules.

## Description

La présente invention concerne un équipement multi-modulaire de traitement d'une région externe du corps d'un être vivant à usage médical ou cosmétique. Elle s'applique plus particulièrement mais non exclusivement au traitement de la peau du corps ou du visage d'un patient humain par photothérapie.

Le traitement par photothérapie consiste en l'application d'un rayonnement lumineux présentant un spectre d'émission prédéfini directement sur la région du corps à traiter. Un tel traitement peut être appliqué également en combinaison avec un produit photo-sensibilisant appliqué sur la région à traiter quelques heures avant son exposition au rayonnement lumineux pour augmenter l'efficacité du traitement. On parle alors dans ce cas de photothérapie dynamique (connue également sous l'acronyme anglais PDT « Photo Dynamic Therapy »).

Le rayonnement est de préférence sensiblement monochromatique ou présente un spectre d'émission compris dans une bande étroite de longueurs d'onde. Généralement, le spectre d'émission comprend une longueur d'onde maximale située dans la lumière visible rouge ou encore dans la lumière visible bleue, ces longueurs d'onde ayant démontré une efficacité particulière pour le traitement de certaines affections de la peau.

Il a été ainsi observé au cours de nombreuses études scientifiques, qu'un traitement par photothérapie dans une plage de longueurs d'onde prédéfinie, notamment dans la lumière rouge ou bleue, permet d'agir de façon bénéfique sur de nombreux problèmes cutanés, tels que les cicatrices, les vergetures, l'acné, les rides et ridules, mais également certains cancers cutanés.

Plus particulièrement, lors de l'application du rayonnement sur la peau, notamment dans la lumière rouge et plus particulièrement autour de la longueur d'onde 630 nm, les cellules de la région irradiées vont réagir selon un principe similaire à celui de la photosynthèse des végétaux. La lumière va stimuler les cellules, et plus spécifiquement certaines structures cellulaires de la cellule, telles que les mitochondries.

De façon connue en soi, les mitochondries jouent un rôle particulièrement important dans le processus de respiration cellulaire ainsi que dans le processus de transformation des nutriments en un vecteur énergétique désigné couramment par ATP (acronyme pour Adénosine Tri Phosphate). Dès lors, la stimulation des mitochondries par la lumière rouge contribue à une régénération des cellules grâce à l'amélioration du processus de respiration et d'alimentation des cellules. Une telle régénération cellulaire va se manifester par exemple pour les cellules du visage par une augmentation de la production de collagène et d'élastine et par conséquent une diminution des rides et ridules.

Il est déjà connu dans l'état de la technique, notamment du document US 6 626 932, une installation de traitement d'un patient par photothérapie. Ce document décrit une installation particulièrement complexe et encombrante. L'installation permet de traiter des surfaces larges du corps de l'utilisateur et comprend à cet effet un habitacle dimensionné pour permettre au patient de s'allonger ou de se tenir debout ou encore assis. A cet effet, l'installation peut comporter une couchette ou un siège. L'installation comprend en outre une source lumineuse formée par une série de tubes fluorescents de grandes dimensions.

Or, un tel appareil est particulièrement complexe et encombrant. Il est également complexe à fabriquer. Par ailleurs, du fait que le patient est situé à distance des tubes de lumière, ces derniers doivent fournir un rayonnement puissant ce qui les rend particulièrement consommateurs d'énergie. En outre, cette installation n'est pas adaptée pour traiter des petites surfaces de peau, comme cela est nécessaire par exemple pour traiter une cicatrice.

Par conséquent, il existe un besoin d'un équipement de photothérapie qui puisse s'adapter à la dimension de la zone à traiter de l'utilisateur qu'elle soit large ou au contraire étroite et qui ne présente pas les inconvénients précités de l'installation de l'art antérieur, à savoir son encombrement et sa complexité. En outre, l'équipement doit avoir une fabrication peu coûteuse et avoir une consommation énergétique réduite.

A cet effet, l'invention a pour objet un interconnecteur structurel et électrique de deux modules entre eux d'un équipement multi-modulaire, du type comprenant des moyens de liaison et des moyens de liaison complémentaires aptes à coopérer ensemble pour former une liaison mécanique libérable des deux modules entre eux, **caractérisé en ce que** les moyens de liaison sont électriquement conducteurs pour autoriser le passage d'un courant électrique entre les deux modules **et en ce que** les moyens de liaison et les moyens de liaison complémentaires sont de type à aimantation pour former une liaison magnétique amovible entre les deux modules.

Ceci permet de former une liaison articulée de façon simple. Une telle liaison présente un axe principal d'aimantation permettant une liaison relativement forte selon cet axe mais qui peut être rapidement rompue par un effort de cisaillement selon une direction perpendiculaire à l'axe principal d'aimantation.

L'invention permet ainsi d'obtenir d'une façon générale un équipement de traitement multi-modulaire par assemblage d'une pluralité de modules. Le module fait partie d'une pluralité de modules. La configuration modulaire permet ainsi d'adapter la dimension de la surface à traiter en fonction du besoin thérapeutique ou cosmétique par des interconnecteurs selon l'invention.

Les modules peuvent être réunis et séparés facilement grâce à un ou plusieurs interconnecteurs selon l'invention.

Par ailleurs, comme la liaison est électriquement conductrice, la mise en oeuvre de l'équipement est très simple : aucun branchement électrique additionnel n'est requis lors de l'assemblage des modules entre eux.

Ainsi, du fait que la surface à traiter est en quelque sorte échantillonnée par les modules, il est possible de couvrir des surfaces plus ou moins grandes sans recourir à des installations complexes et encombrantes.

Par liaison mécanique, au sens de l'invention, on entendra la relation des modules entre eux liés par un ou des contacts physiques ou directs qui les rendent totalement ou partiellement solidaires.

Un interconnecteur selon l'invention peut comporter l'une ou l'autre des caractéristiques suivantes :
- les moyens de liaison et les moyens de liaison complémentaires sont magnétiques de polarité opposée ;
- les moyens de liaison et les moyens de liaison complémentaires comprennent chacun au moins un élément de liaison magnétique dit « individuel » et l'un des moyens de liaison et des moyens de liaison complémentaires comprennent un élément de liaison magnétique dit « mutuel » de forme générale sphérique, cet élément mutuel sphérique étant entièrement séparable des deux modules ;
- l'élément de liaison « individuel » a une forme générale de pastille et l'élément de liaison « mutuel » a une forme générale de bille ;
- la liaison mécanique des moyens de liaison et des moyens de liaison complémentaires est de type sphère-plan, sphère-sphère ou sphère-creux ;
- les moyens de liaison sont réalisés dans des matériaux comprenant essentiellement des terres rares ;
- l'élément mutuel comprend un corps sphérique aimanté et est recouvert d'une pellicule réalisée dans un matériau comprenant un alliage à base d'or.

L'invention a encore pour objet un équipement multi-modulaire comprenant au moins deux modules, chaque module étant du type comprenant un boîtier propre à être saisi par un utilisateur comprenant un organe fonctionnel électrique, **caractérisé en ce qu'i**l comprend un interconnecteur selon l'invention pour former une liaison structurelle et électrique des deux modules entre eux.

Dans un mode de réalisation préféré de l'invention, l'équipement comprend un premier interconnecteur et un second interconnecteur qui définissent entre eux un axe, la liaison mécanique entre les deux modules forme un pivot le long de cet axe.

Dans un mode de réalisation préféré de l'invention, le premier interconnecteur comprenant des premiers moyens de liaison et des premiers moyens de liaison complémentaires et le second interconnecteur comprenant des seconds moyens de liaison et des seconds moyens de liaison complémentaires, les premiers moyens de liaison et les seconds moyens de liaison du module sont complémentaires l'un de l'autre et sont magnétiques de polarité opposée.

De préférence, l'organe fonctionnel électrique de chaque module est choisi parmi une batterie d'alimentation et un organe de traitement à usage médical ou cosmétique du type à émission de lumière.

De préférence, l'organe fonctionnel comprend une source lumineuse comprenant une pluralité de diodes électroluminescentes et le boîtier comprend une fenêtre d'émission de la lumière émise par la source.

De préférence, la liaison mécanique des moyens de liaison et des moyens de liaison complémentaires est de type sphère-plan, sphère-sphère ou sphère-creux.

De préférence, le boîtier comprend des premiers et seconds moyens de liaison aptes à coopérer respectivement avec des premiers et deuxièmes moyens complémentaires respectivement d'un premier autre module et d'un deuxième autre module, les premiers et seconds moyens étant agencés sur deux côtés opposés du boîtier pour le raccordement latéral du module avec les premier et deuxième autres modules.

De préférence, les premiers moyens de liaison et les seconds moyens de liaison du module sont complémentaires l'un de l'autre et sont magnétiques de polarité opposée.

Ceci permet d'assurer la complémentarité de deux modules entre eux de la pluralité de modules. Lorsque les modules identiques entre eux sont mis à proximité les uns des autres, par définition, les premiers moyens de liaison de l'un des modules sont complémentaires des seconds moyens de liaison de l'autre des modules.

D'autres caractéristiques et avantages de l'invention apparaîtront à la lumière de la description qui suit, faite en référence aux dessins annexés dans lesquels :
- la figure 1 représente une vue en perspective d'un équipement de photothérapie selon un premier mode de réalisation de l'invention;
- la figure 2 représente une vue partielle de face d'une pluralité de modules de photothérapie de l'équipement de la figure 1 ;
- la figure 3 représente une vue de dessus d'un module de traitement de la pluralité de modules représentée sur la figure 2 ;
- la figure 4 représente une vue en perspective et en coupe selon la ligne 4-4 de la figure 3 ;
- la figure 5 représente une vue éclatée du module de traitement de la figure 3 ;
- la figure 6 représente une vue éclatée d'un module d'alimentation électrique de la pluralité de modules représentée sur la figure 2 ;
- la figure 7 représente une vue en perspective d'une partie intérieure d'un boîtier du module de la figure 5 ou de la figure 6 ;
- les figures 8A et 8B représentent des vues schématiques de détail de moyens de liaisons libérables de deux modules de la figure 2 ;

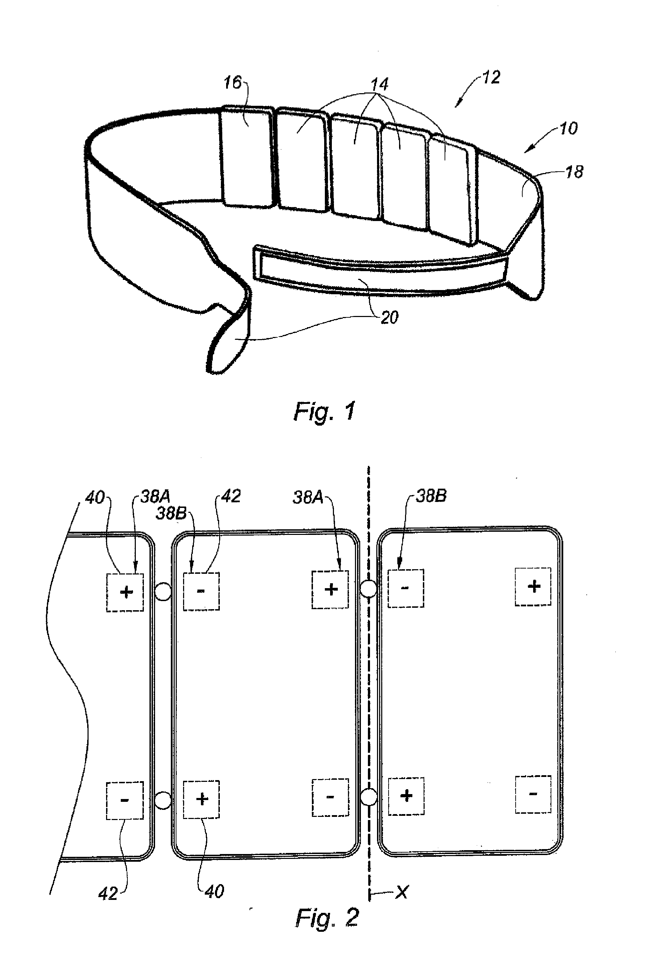

On a représenté de façon schématique sur **la** **figure 1** un équipement de traitement par photothérapie selon un premier mode de réalisation de l'invention. Cet équipement est désigné par la référence générale 10.

Cet équipement 10 comprend une pluralité 12 de modules portatifs selon l'invention. La pluralité 12 de modules comprend au moins un module 14 de traitement d'une zone externe d'un utilisateur par exemple par photothérapie, dit module lumineux, et au moins un module 16 d'alimentation électrique de ce module lumineux 14. At cet effet, chaque module 14 ou 16 comprend un organe fonctionnel électrique adapté au traitement ou à l'alimentation électrique de l'équipement 10. Dans l'exemple illustré par la **figure 1**, la pluralité 12 de modules comprend quatre modules lumineux 14 et un module d'alimentation électrique 16 des quatre modules lumineux 14. Bien entendu, en variante, l'équipement peut comporter des modules de traitement autres que par l'émission d'un flux lumineux.

L'équipement 10 comprend également dans l'exemple décrit une bande 18 formant support des modules 14, 16. La bande 18 comprend des moyens 20 d'adaptation de la dimension de la bande 18 à la dimension de la partie du corps de l'utilisateur. Les moyens d'adaptation 20 comprennent par exemple des moyens de fermeture choisis parmi des moyens de fermeture du type auto-agrippant, des moyens de fermeture à pression et des moyens de fermeture à boucle/crochet. Dans le cas présent, les moyens de fermeture sont de type auto-agrippant.

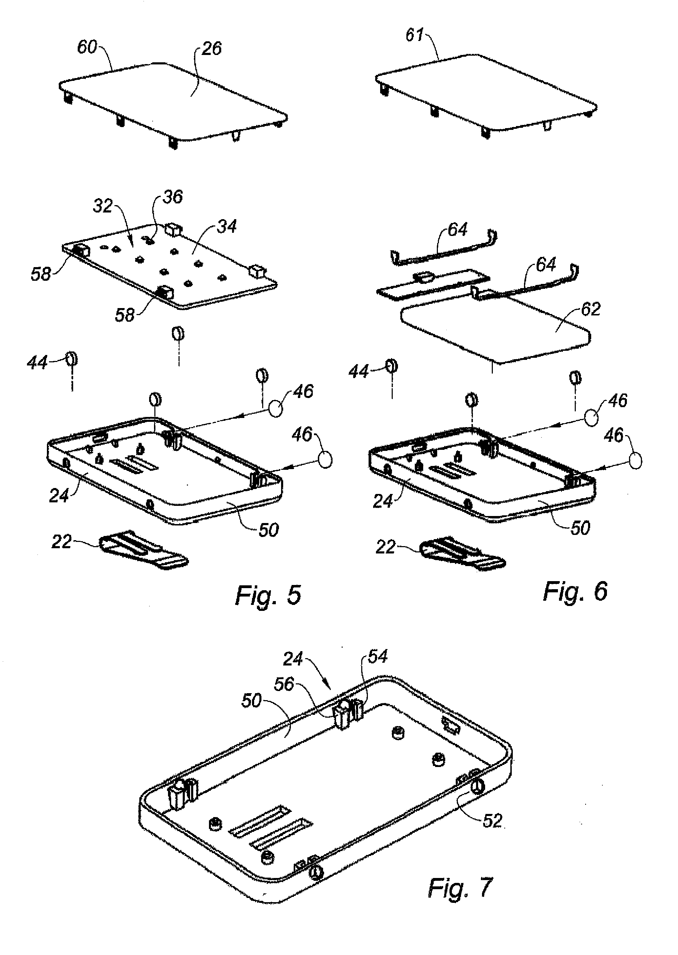

Par ailleurs, de préférence, les modules 14, 16 comprennent des moyens 22 de fixation à la bande 18 de support. Ces moyens de fixation 22 comprennent par exemple des éléments forment pince ou « clip ». Un tel élément 22 est illustré par exemple sur **les** **figures 5 et 6**.

On va maintenant décrire plus en détail en référence aux **figures 2 à 5**, un mode de réalisation préféré d'un module lumineux 14 selon l'invention. On ne décrira par la suite qu'un seul module lumineux car les quatre modules lumineux sont identiques entre eux.

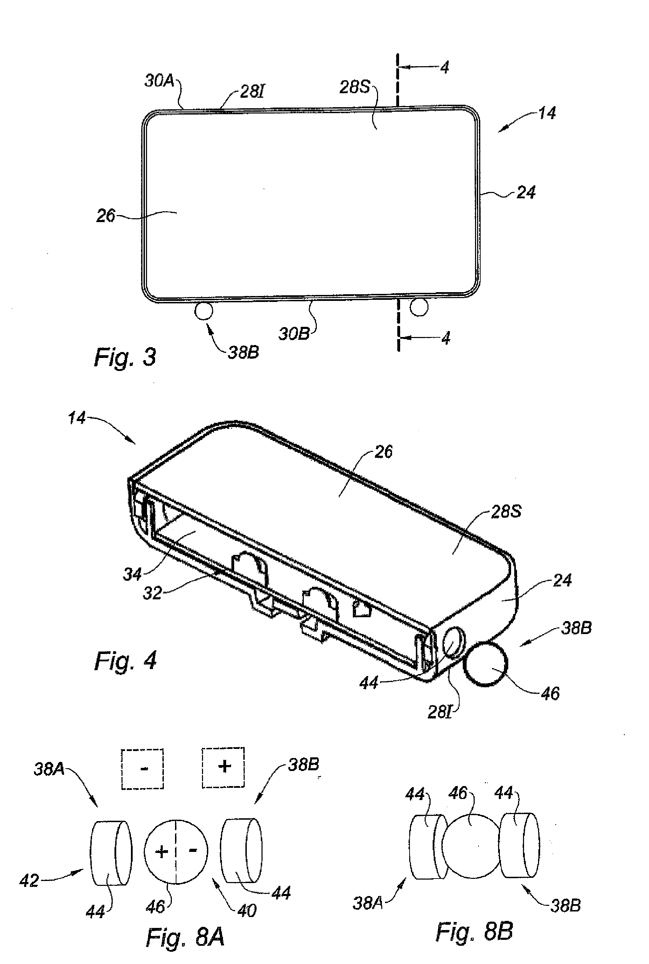

Selon l'invention, le module 14 comprend un boîtier 24 propre à être saisi par l'utilisateur. Comme cela est illustré sur **la** **figure 3**, le boîtier 24 du module lumineux 14 a une forme générale parallélépipédique délimitant une face supérieure 28S (ou avant) et une face inférieure 28I (ou arrière) et deux côtés opposés 30A, 30B. Dans l'exemple illustré, la face inférieure 28I porte les moyens de fixation 22 du module 14 à la ceinture 18. Ce boîtier 24 est muni d'une fenêtre 26 d'émission d'un flux lumineux. Dans l'exemple décrit, la fenêtre 26 est réalisée dans un matériau translucide, par exemple un matériau plastique tel que du polycarbonate. De préférence, la fenêtre 26 est formée sur une des faces du boîtier 24, par exemple sur la face avant 28S.

En outre, le module lumineux 14 comprend une source 32 de génération d'un flux lumineux à travers la fenêtre 26. Comme cela est illustré par **les** **figures 4** **et** **5**, la source de lumière 32 est portée par un support 34. Par exemple, ce support 34 forme un support pour un circuit imprimé comprenant notamment un circuit d'alimentation électrique de la source 32.

De préférence et comme cela est illustré sur **la** **figure 5**, la source de lumière 32 comprend une pluralité d'unités 36 de génération d'un flux lumineux. Chaque unité 36 comprend de préférence un composant électronique de type à diode électroluminescente (désigné ci-après par l'acronyme DEL). Par exemple, le composant électronique comprend de façon classique un boîtier de composant, une embase formant support et au moins une puce semiconductrice DEL portée par l'embase et des pattes de connexion électrique de la puce à des moyens externes tels qu'au circuit électrique d'alimentation de la source de lumière 32. Ce composant peut comporter en outre un système optique permettant d'extraire le flux lumineux produit par la puce semiconductrice. Le composant est par exemple de type composant monté en surface. Ainsi, dans l'exemple illustré sur la **figure 5**, les composants électroniques DEL sont montés en surface sur le support 34 de circuit imprimé.

De préférence, la source lumineuse 32 a un spectre d'émission présentant une longueur d'onde d'émission maximale comprise dans la plage de longueurs d'onde choisies parmi les plages 400 nm à 440 nm, 610 nm à 650 nm et 810 nm à 850 nm.

Par ailleurs, le module lumineux 14 comprend en outre de préférence des moyens de pilotage (non représentés) de ces unités lumineuses 36 selon un programme de traitement de photothérapie prédéfini. Ce programme de traitement peut par exemple être mémorisé par les moyens de pilotage.

Le programme de traitement comprend par exemple des instructions de code pour la réalisation d'étapes choisies parmi une étape d'allumage de la source 32, une étape d'extinction de la source 32, une étape de définition d'un mode d'allumage pulsé ou continu de la source 32, une étape de définition d'une durée de période d'allumage de la source 32.

Par exemple, les moyens de pilotage des unités 36 comprennent un microcontrôleur porté par le support 34, apte à mémoriser le programme de pilotage.

De préférence, les unités 36 de la source 32 de lumière sont raccordées électriquement en série entre elles. Ceci permet avantageusement de limiter l'intensité du courant circulant dans le circuit d'alimentation électrique de la source de lumière 32 et ne requérir que seulement deux bornes pour l'alimentation de la source 32.

Plus particulièrement, selon l'invention, le module lumineux 14 comprend des moyens 38A de liaison du module 14 aptes à coopérer avec des moyens de liaison 38B complémentaires d'un autre module 14 ou 16. De façon plus précise et conformément à l'invention, ces moyens de liaison 38A, 38B sont agencés pour permettre une liaison mécanique articulée libérable de deux modules 14 entre eux. Par ailleurs, ces moyens de liaison 38A, 38B sont électriquement conducteurs pour autoriser le passage d'un courant électrique entre les deux modules 14. Les moyens de liaison 38A et 38B forment un interconnecteur structurel et électrique.

De préférence, les moyens de liaison 38A et les moyens de liaison complémentaires 38B sont de type à aimantation pour former une liaison magnétique amovible entre les deux modules de la pluralité de modules.

De préférence, chaque module 14 comprend des premiers 38A et seconds 38B moyens de liaison aptes à coopérer respectivement avec des premier 38B et deuxième 38A moyens de liaison complémentaires respectivement d'un premier autre module 14 et d'un deuxième autre module 14. Dans l'exemple décrit, les premiers 38A et deuxièmes 38B moyens de liaison sont agencés sur deux côtés opposés 30A, 30B du boîtier 24 pour le raccordement latéral du module 14 avec les deux autres modules, comme cela est illustré par **la** **figure 2**.

De préférence, les premiers 38A et seconds 38B moyens de liaison du module 14 sont complémentaires l'un de l'autre et sont magnétiques de polarité opposée. De ce fait, lorsque deux modules lumineux 14 sont présentés à proximité l'un de l'autre, les premiers moyens de liaison 38A du premier module 14 sont aptes à coopérer avec les seconds moyens de liaison 38B du deuxième module 14 par attraction magnétique puisque par définition, les premiers moyens de liaison 38A sont complémentaires des seconds moyens de liaison 38B d'un même module.

Comme illustré sur **cette** **figure 2**, les premiers moyens de liaison 38A comprennent une paire d'éléments de liaison 40 et 42 et les moyens de liaison 38B comprennent une paire d'éléments de liaison complémentaires 40, 42. Les éléments de liaison 40 et 42 de chaque paire 38A, 38B sont magnétiques de polarité opposée afin de définir un unique sens de montage des deux modules entre eux.

Toutefois, en variante, les éléments de liaison de chaque paire 38A, 38B peuvent être de polarité identique pour une même paire et ce sont les deux paires 38A, 38B des premiers moyens et des seconds moyens de liaison de chaque module qui ont des polarités opposées.

Bien entendu, en variante, seulement l'un des moyens de liaison 38A, 38B peut être de type aimanté, tandis que l'autre des moyens de liaison complémentaires 38B, 38A peut être de type aimantable, c'est-à-dire être réalisé dans un matériau métallique apte à créer une liaison magnétique avec un aimant. Toutefois, de préférence, les deux moyens de liaison 38A, 38B sont aimantés pour assurer une liaison forte.

Par exemple, les moyens de liaison sont réalisés dans un matériau comprenant des métaux terres rares telles qu'un métal de la famille des Lanthanides. Par exemple, un matériau aimanté à base de Néodyme présente de nombreux avantages : force d'attraction élevée, faible encombrement, poids réduit, aspect esthétique améliorée. Toutefois, en variante, les moyens de liaison peuvent être réalisés dans un matériau ferromagnétique tel que du fer, du cobalt ou encore du nickel.

De préférence, les moyens de liaison 38A et les moyens de liaison complémentaires 38B comprennent au moins un élément de liaison magnétique dit « individuel » 44. On a ainsi représenté en détail sur les figures **8A, 8B****,** deux éléments de liaison complémentaires 40, 42 respectivement d'une paire de moyens de liaison 38A et d'une paire de moyens de liaison complémentaires 38B.

Ainsi, de préférence, l'élément de liaison magnétique 44 est formé par un premier aimant comprenant un pôle mâle et un pôle femelle. Dans l'exemple décrit, ce premier aimant 44 a une forme générale de pastille.

On entend par pastille au sens de l'invention, une pièce en trois dimensions dont la largeur et la longueur sont du même ordre de grandeur, cet ordre de grandeur étant bien supérieur à celui de la hauteur. Cette pastille peut ainsi présenter une face supérieure ou inférieure plate, bombée, concave ou convexe et peut indifféremment être de forme générale ovale, ronde, rectangulaire, octogonale, polygonale, etc.

Dans l'exemple décrit, les premiers aimants 44 sont destinés à être logés à l'intérieur du boîtier 24 des modules 14 et sont agencés pour présenter une face magnétique accessible de l'extérieur comme illustré sur **la** **figure 4**.

On a illustré en détail sur **la** **figure 7** une partie du boîtier 24. Ce boîtier 24 comprend une coque 50 munie d'ouvertures 52. En regard de chaque ouverture 22 de la coque 50 est ménagé un logement 54 apte à recevoir chacun des éléments individuels 44. Le logement 54 est agencé de manière à ce que les aimants 44 puissent être accessibles de l'intérieur et de l'extérieur de la coque 50 à travers les ouvertures 52. Ceci permet de réaliser un raccordement électrique à la fois à l'intérieur du boîtier 24 avec notamment la source de lumière 32 mais également à l'extérieur du boîtier 24 avec un autre module 14.

Le logement 54 est formé par une encoche ouverte définie par deux rails de guidage 56 de l'aimant 44 à l'intérieur de l'encoche.

Dans l'exemple décrit, le boîtier 24 comprend en outre un couvercle 60 portant la fenêtre d'émission 26 comme cela est illustré sur **la** **figure 5**. De préférence, lors de la fermeture du boîtier 24 par assemblage de la coque 50 avec le couvercle 60, les éléments 44 sont maintenus mécaniquement dans leur logement 54 par le couvercle 60. Par conséquent, ces éléments individuels 44 ne peuvent être séparés du module 14 une fois le boîtier 24 fermé.

Par ailleurs, le module lumineux 14 comprend des contacteurs électriques 58 aptes à créer une connexion électrique entre le circuit imprimé porté par le support 34 et les moyens de liaison 38A, 38B. Dans l'exemple illustré, les contacteurs 54 sont formés par des plots portés par le support 34. Ces plots comprennent par exemple une broche métallique apte à se déformer contre les moyens de liaisons 38A, 38B lors de l'introduction du support 34 dans le boîtier 24.

En outre, selon un mode de réalisation préféré de l'invention, l'un des moyens de liaison 38A, 38B et des moyens de liaison complémentaires 38B, 38A comprennent un élément 46 de liaison magnétique dit « mutuel » de forme générale sphérique. De préférence, cet élément mutuel sphérique 46 est entièrement séparable des deux modules 14. De préférence, cet élément mutuel sphérique 46 est formé par un deuxième aimant. Cet élément mutuel 46 a une forme générale de bille.

Cet élément mutuel 46 comprend un corps sphérique aimanté est recouvert d'une pellicule réalisée dans un matériau comprenant un alliage à base d'or. L'alliage comprend par exemple du nickel, du cuivre et de l'or. La pellicule a dans cet exemple une épaisseur d'environ 3 microns et est par exemple déposée par électrolyse. L'ajout d'une couche à base d'or en surface de l'élément mutuel permet d'obtenir des propriétés électriquement conductrices améliorées par rapport à un élément mutuel 46 à base uniquement de nickel.

Cet élément de liaison 46 est dit « mutuel » car il peut indifféremment se fixer sur l'un ou l'autre module par attraction magnétique sur l'élément individuel 44 de l'un ou l'autre des modules. Cette désignation « mutuel » est à opposer à la désignation « individuel ».

Comme illustré en détail par **les** **figures 8A, 8B**, de préférence, l'élément individuel 44 est apte à coopérer avec l'élément mutuel 46 selon une liaison mécanique de type sphère-plan. Bien entendu, dans des variantes non illustrées sur les figures, les moyens de liaison et les moyens de liaison complémentaires peuvent coopérer selon une liaison mécanique sphère-sphère ou sphère-creux. Dans ce cas par exemple, l'élément individuel 44 peut comporter une face creuse aimantée apte à recevoir l'élément mutuel sphérique 46 ou encore une face bombée apte à coopérer avec l'élément mutuel sphérique 46 selon une liaison sphère-sphère.

Dans le mode de réalisation préféré de l'invention, selon **la** **figure 2**, les moyens de liaison 38A, 38B définissent un axe X passant par la paire d'éléments de liaison 40, 42. La liaison mécanique formée entre les deux modules forme un pivot le long de cet axe X. Une telle liaison permet une flexibilité des modules entre eux par articulation des modules 14 entre eux.

De préférence, afin de réaliser cette fonction de sécurité, le module lumineux comprend un capteur tactile et une surface tactile liée au capteur (non représentés sur les figures). De préférence, la surface tactile s'étend au moins partiellement sur la fenêtre d'émission.

Un capteur tactile, dit également capacitif, permet notamment de détecter si une personne touche ou effleure un objet. Un tel capteur comporte une ou plusieurs électrodes reliées à un circuit de détection de la capacitance de la ou des électrodes. Lorsqu'une personne touche ou effleure l'électrode, la capacitance de l'électrode charge et se trouve détectée par le circuit de détection qui est apte à délivrer un signal de sortie représentant l'information.

L'équipement 10 selon l'invention comprend également un module d'alimentation électrique 16.

Selon l'invention, le module d'alimentation 16 comprend un boîtier propre 24 à être saisi par un utilisateur. Ce boîtier 24 a une forme générale identique à celle du boîtier 24 des modules lumineux 14.

Par ailleurs, le module d'alimentation 16 comprend une source d'alimentation électrique 62 raccordée électriquement à des bornes électriques accessibles de l'extérieur du boîtier 24. Plus précisément, selon l'invention, les bornes électriques forment des moyens de liaison complémentaires 38A, 38B des moyens de liaison 38B, 38A du module lumineux 14. Enfin, les moyens de liaison complémentaires sont agencés pour permettre une liaison électriquement conductrice mécanique libérable entre le module d'alimentation 16 et l'un au moins des modules lumineux 14.

Ainsi, comme cela est illustré sur **les** **figures 1 et 2**, le module d'alimentation 16 et le module lumineux 14 sont aptes à créer une liaison magnétique amovible de façon identique à la liaison magnétique amovible créée entre deux modules lumineux 14 telle que décrite ci-dessus. On ne décrira par conséquent pas plus les moyens de liaison 38A, 38B du module d'alimentation électrique 16 qui sont analogues à ceux déjà décrits en rapport aux modules lumineux 14.

La source d'alimentation électrique 62 est par exemple une batterie portative. Afin de recharger la batterie, le module d'alimentation 16 comprend une prise électrique. Par ailleurs, dans l'exemple, le module d'alimentation 16 comprend des circuits électriques diverses pour connecter la batterie aux bornes électriques d'alimentation.

On a illustré sur **les** **figures 5 et 6** une vue éclatée du module d'alimentation 16 selon ce premier mode de réalisation. On voit notamment que ce module d'alimentation 16 comprend quatre aimants 44 formant les éléments individuels agencés dans la coque 50 du boîtier 24.

Pour fabriquer le module lumineux 14 ou le module d'alimentation 16 selon le premier mode, on procède à un certain nombre d'étapes identiques. En effet, le boîtier des modules 14 et 16 est formé par assemblage d'une même coque 50 et d'un même couvercle 60 délimitant entre eux un volume intérieur fermé.

Initialement, on fait une première étape d'agencement des moyens de liaison 38A, 38B à l'intérieur de la coque 50 de manière à ce qu'ils puissent être accessibles de l'intérieur et de l'extérieur de la coque 50 à travers les ouvertures 52 ménagées dans la coque 50.

On agence l'organe principal du module 14 ou 16 à savoir, la source de lumière 32 pour le module lumineux 14 et la batterie 62 pour le module d'alimentation 16.

Dans le mode de réalisation de l'invention, lors de la fabrication du module lumineux 14, on introduit en force le support 34 dans la coque 50 afin d'établir un contact électrique des contacteurs avec les moyens de liaison 38A, 38B par déformation des broches métalliques contre les moyens de liaison 38A, 38B.

Lors de la fabrication du module d'alimentation 16, on agence la batterie 62 à l'intérieur de la coque 50 ainsi que des languettes électriquement conductrices 64 traversant la coque dans une direction transversale pour connecter électriquement les premiers 38A et seconds 38B moyens de liaison entre eux et former les bornes d'alimentation électrique du module 16.

Au cours d'une dernière étape commune à la fabrication des deux modules 14 et 16, on ferme le boîtier 24 par encliquetage de la coque 50 et respectivement du couvercle 60 comprenant la fenêtre 26 et du couvercle 61 de préférence sans fenêtre.

Dans ce premier mode de réalisation correspondant à la première utilisation illustrée par **les** **figures 1, 2**, les modules 14, 16 sont assemblés latéralement et sont portés par le support côte à côte. Ceci permet ainsi de couvrir une surface de traitement de dimensions importantes.

De ce qui précède, il ressort que l'invention remédie bien aux inconvénients signalés au début en les supprimant.

Bien entendu, d'autres modes de réalisation sont envisageables sans sortir du cadre de l'invention. Ainsi, diverses modifications peuvent être apportées par l'homme du métier à l'invention qui vient d'être décrite à titre d'exemple.

## Revendications

1. Interconnecteur structurel et électrique de deux modules (14, 16) entre eux d'un équipement multi-modulaire, du type comprenant des moyens de liaison (38A) d'un premier module (14, 16) et des moyens de liaison complémentaires (38B) d'un second module (14, 16) aptes à coopérer ensemble pour former une liaison mécanique libérable des deux modules (14, 16) entre eux, **caractérisé en ce que** les moyens de liaison (38A, 38B) sont électriquement conducteurs pour autoriser le passage d'un courant électrique entre les deux modules (14,16) **et en ce que** les moyens de liaison (38A, 38B) sont de type à aimantation pour former une liaison magnétique amovible entre les deux modules (14, 16).

2. Interconnecteur selon la revendication précédente, dans lequel les moyens de liaison (38A) et les moyens de liaison complémentaires (38B) sont magnétiques de polarité opposée.

3. Interconnecteur selon la revendication précédente, dans lequel les moyens de liaison (38A, 38B) sont réalisés dans des matériaux comprenant essentiellement des terres rares, tel que du Néodyme.

4. Interconnecteur selon l'une quelconque des revendications précédentes, dans lequel les moyens de liaison (38A) et les moyens de liaison complémentaires (38B) comprennent chacun au moins un élément de liaison magnétique (44) dit « individuel » et l'un des moyens de liaison (38A) et des moyens de liaison complémentaires (38B) comprennent un élément de liaison magnétique (46) dit « mutuel » de forme générale sphérique, cet élément mutuel sphérique (46) étant entièrement séparable des deux modules (14, 16).

5. Interconnecteur selon la revendication précédente, dans lequel l'élément de liaison « individuel » a une forme générale de pastille et l'élément de liaison « mutuel » a une forme générale de bille.

6. Interconnecteur selon l'une quelconque des revendications 3 à 5, dans lequel l'élément mutuel comprend un corps sphérique aimanté et est recouvert d'une pellicule réalisée dans un matériau comprenant un alliage à base d'or.

7. Equipement multi-modulaire (10) comprenant au moins deux modules (14, 16), chaque module (14, 16) étant du type comprenant un boîtier (24) propre à être saisi par un utilisateur comprenant un organe fonctionnel électrique, **caractérisé en ce qu'**il comprend un interconnecteur selon l'une quelconque des revendications précédentes pour former une liaison structurelle et électrique des deux modules (14, 16) entre eux.

8. Equipement (10) selon la revendication précédente, comprenant un premier interconnecteur et un second interconnecteur qui définissent entre eux un axe, la liaison mécanique entre les deux modules (14, 16) forme un pivot le long de cet axe.

9. Equipement (10) selon la revendication précédente, dans lequel, le premier interconnecteur comprenant des premiers moyens de liaison (38A) et des premiers moyens de liaison complémentaires (38B) et le second interconnecteur comprenant des seconds moyens de liaison (38B) et des seconds moyens de liaison complémentaires, les premiers moyens de liaison (38A) et les seconds moyens de liaison (38B) du module (14) sont complémentaires l'un de l'autre et sont magnétiques de polarité opposée.

10. Equipement (10) selon l'une quelconque des revendications 7 à 9, dans lequel l'organe fonctionnel électrique de chaque module est choisi parmi une batterie d'alimentation et un organe de traitement à usage médical ou cosmétique du type à émission de lumière.
